(19) Europäisches Patentamt
European Patent Office
Office européen des brevets

(11) Veröffentlichungsnummer: **0 025 859**
**A2**

(12) # EUROPÄISCHE PATENTANMELDUNG

(21) Anmeldenummer: **80104830.7**

(22) Anmeldetag: **14.08.80**

(51) Int. Cl.³: **C 07 D 249/08**

(30) Priorität: **31.08.79 DE 2935164**

(43) Veröffentlichungstag der Anmeldung:
**01.04.81 Patentblatt 81/13**

(84) Benannte Vertragsstaaten:
**AT BE CH DE FR GB IT LI LU NL SE**

(71) Anmelder: **BASF Aktiengesellschaft**
**Carl-Bosch-Strasse 38**
**D-6700 Ludwigshafen(DE)**

(72) Erfinder: **Jacobs, Peter, Dr.**
**Kalmitstrasse 2**
**D-6718 Gruenstadt(DE)**

(72) Erfinder: **Mangold, Dietrich, Dr.**
**Hermann-Walker-Strasse 49**
**D-6903 Neckargemuend(DE)**

(72) Erfinder: **Oeser, Heinz-Guenter, Dr.**
**Friedrich-Burschell-Weg 19**
**D-6700 Ludwigshafen(DE)**

(54) **Verfahren zur Herstellung von 1,2,4-Triazolen.**

(57) Herstellung von 1,2,4-Triazolen durch Umsetzung von 3-Amino-1,2,4-triazolen mit Alkoholen und Nitrosierungsmitteln in Gegenwart von Wasser und Schwefelsäure. Die nach den Verfahren der Erfindung herstellbaren neuen Verbindungen sind wertvolle Ausgangsstoffe für die Herstellung von Pharmazeutika, Farbstoffen und Schädlingsbekämpfungsmitteln.

EP 0 025 859 A2

Croydon Printing Company Ltd.

# Verfahren zur Herstellung von 1,2,4-Triazolen

Die Erfindung betrifft ein neues Verfahren zur Herstellung von 1,2,4-Triazolen durch Umsetzung von 3-Amino-1,2,4-triazolen mit Alkoholen und Nitrosierungsmitteln in Gegenwart von Wasser und Schwefelsäure.

Es ist aus J. Amer. Chem. Soc., Band 76, Seiten 290 bis 291 (1954) bekannt, daß man Tetrazol nur über sein Tetrazoldiazoniumsalz erhalten kann, wenn man die Diazotierung von 5-Aminotetrazol in Gegenwart von unterphosphoriger Säure durchführt. Es wird ausdrücklich vor der Gefährlichkeit des extrem explosiven Diazoniumsalzes gewarnt und eine sichere Verfahrensweise nur in der Verwendung dieser Säure gesehen. Die gleiche Verfahrensweise wird aus demselben Grunde auch für die Herstellung von 1,2,4-Triazol durch Desaminierung von 3-Amino-1,2,4-triazol empfohlen und eine Ausbeute von 75,3 Prozent erhalten. Eine Arbeit in Annalen, Band 273 (1893), Seite 144, weist in diesem Zusammenhang darauf hin, daß Aminotetrazol als Diazoverbindung in wäßriger Lösung bei 0°C von selbst explodiert.

Es ist aus der Zeitschrift für Chemie, Band 9 (1969), Seite 332, bekannt, daß man Hydrazinhydrat und 85-prozentige Ameisensäure auf 200°C erhitzt, das Gemisch durch Abkühlen zum Erstarren bringt und in wäßriger, salzsaurer Lösung mit Natriumnitrit umsetzt. Man erhält im

WB/BL

industriellen Maßstab 1,2,4-Triazol in 55- bis 60-prozentiger Ausbeute.

Ebenfalls ist bekannt (J. Chem. Soc., Band 109 (1916), Seiten 155 bis 160), daß 5-Amino-1,2,4-triazol, Salzsäure und Natriumnitrit oder Äthylnitrit nicht zu 1,2,4-Triazol, sondern zu 5-Chlor-1,2,4-triazol umgesetzt werden. Führt man die Umsetzung mit Salpetersäure durch und setzt alkalische ß-Naphthollösung hinzu, erhält man 1,2,4-Triazol-5-azo-ß-naphthol. Analog erfolgt die Herstellung von 3-Methyl-1,2,4-triazol-5-azo-ß-naphthol und 1,2,4-Triazol-5-azo-ß-naphthylamin mit Naphthylamin als Azokomponente. Setzt man 5-Amino-3-methyl-1,2,4-triazol mit Äthylnitrit und alkoholischer Salzsäure um, erhält man ebenfalls das 5-Chlor-3-methyl-1,2,4-triazol. Die Autoren der Arbeit weisen auf die Instabilität der Diazoverbindung von Aminotriazolen unter Bildung von Chlortriazolen hin. Kristallisiert man 3-Methyl-1,2,4-triazol-5-isodiazohydroxid aus Äthylalkohol um, so lagert sich nach Meinung der Autoren ein Molekül Alkohol an diese Verbindung, die sich nach Meinung der Autoren bei Lösung in kalter, konzentrierter Schwefelsäure in das normale Diazoniumsalz verwandelt. Es wird darauf hingewiesen, daß ein so gebildetes Diazoniumsalz, auch in schwefelsaurem Medium, mit alkoholischer Naphthollösung zum 3-Methyl-1,2,4-triazol-5-azo-ß-naphthol kuppelt.

Es wurde nun gefunden, daß man in 1-, 2- und 4-Stellung unsubstituierte, in 3- und/oder 5-Stellung gegebenenfalls durch jeweils einen aliphatischen oder aromatischen Rest substituierte 1,2,4-Triazole durch Desaminierung von Aminotriazolen unter Verwendung von Nitrosierungsmitteln und Säure vorteilhaft erhält, wenn man gegebenenfalls in 3- und/oder 5-Stellung durch jeweils einen aliphatischen oder aromatischen Rest substituierte

Amino-1,2,4-triazole mit aliphatischen Alkoholen und Nitrosierungsmitteln in Gegenwart von Wasser und Schwefelsäure umsetzt.

Die Umsetzung kann für den Fall der Verwendung von 3-Amino-1,2,4-triazol, Natriumnitrit, Schwefelsäure und Äthanol durch die folgenden Formeln wiedergegeben werden:

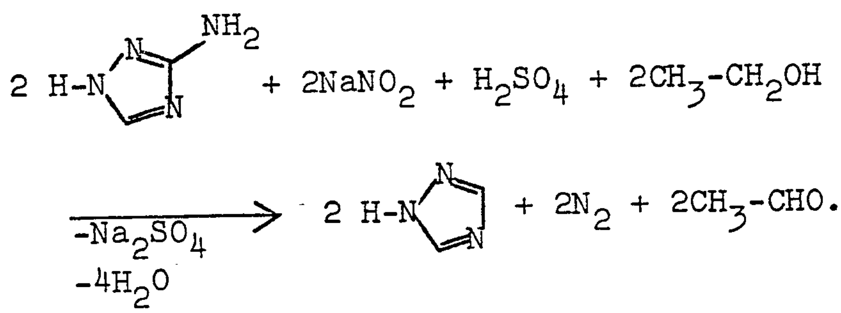


Im Hinblick auf den Stand der Technik liefert das Verfahren nach der Erfindung auf einfacherem und wirtschaftlicherem Wege 1,2,4-Triazole in besserer Ausbeute und Reinheit, gerade auch im industriellen Maßstab. Die Verwendung der schwer zugänglichen unterphosphorigen Säure und damit eine Umweltbelastung mit Phosphaten werden vermieden. Das Verfahren ist im Hinblick auf alle Diazoverbindungen als Ausgangsstoff verwendende Verfahren betriebssicherer, insbesondere ist auch ihre Übertragung in den großtechnischen Maßstab gegeben, da keine Neigung des Reaktionsgemischs zu explosiven Zersetzungen zu beobachten ist. Die Bildung größerer Mengen an möglicherweise sicherheitstechnisch bedenklichen Diazonium-1,2,4-triazolen wird vermieden. Alle diese erfindungsgemäßen Vorteile, gerade auch ohne Verwendung von unterphosphoriger Säure, sind im Hinblick auf den Stand der Technik überraschend. Insbesondere hätte man die Herstellung von heterogenen Gemischen an Zersetzungsprodukten, Diazoniumverbindungen, Azoverbindungen, Sulfotriazolen, oder anderen Stickstoffverbindungen erwarten müssen.

Mit dem erfindungsgemäßen Verfahren werden 1,2,4-Triazole der Formel

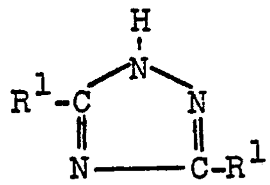

I,

worin die einzelnen Reste $R^1$ gleich oder verschieden sein können und jeweils ein Wasserstoffatom oder einen aliphatischen oder aromatischen Rest bedeuten, hergestellt; sie sind mit den 2,1,4-Triazolen der Formel

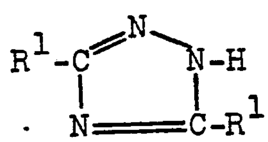

Ia

und den 4,2,1-Triazolen der Formel

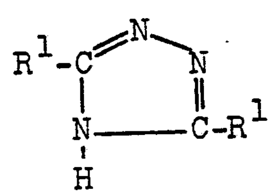

Ib,

worin $R^1$ die vorgenannte Bedeutung besitzt, tautomer und somit auch die Endstoffe Ia und Ib durch das erfindungsgemäße Verfahren herstellbar. Als Ausgangsstoffe kommen 3-Amino-1,2,4-triazole der Formel

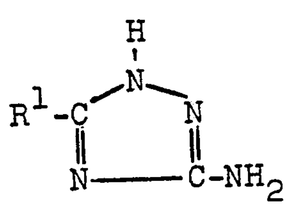

IIa,

und 5-Amino-1,2,4-triazole der Formel

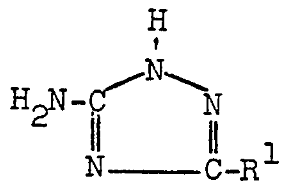 IIb,

die zueinander und zu den entsprechenden 3-Amino-2,1,4-triazolen und 5-Amino-2,1,4-triazolen der Formel

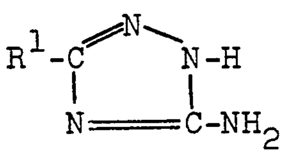 IIc und 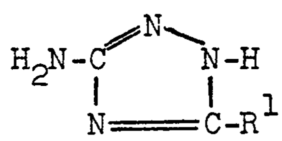 IId

und zu den entsprechenden 3-Amino-4,2,1-triazolen und 5-Amino-4,2,1-triazolen der Formel

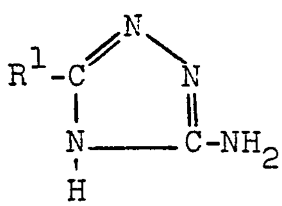 IIe und 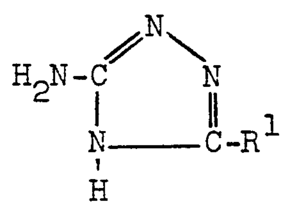 IIf

tautomer sind, und somit allgemein C-Amino-1,2,4-triazole der Formel

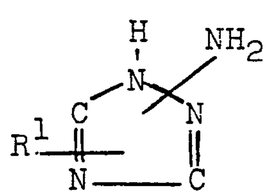 II,

worin die einzelnen Reste $R^1$ gleich oder verschieden sein können und jeweils ein Wasserstoffatom oder einen aliphatischen oder aromatischen Rest bedeuten, in Betracht.

Weiterhin kommen als Ausgangsstoffe 1-Amino-1,2,4-triazole der Formel

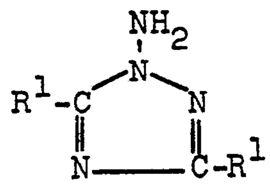

IIIa,

2-Amino-2,1,4-triazole der Formel

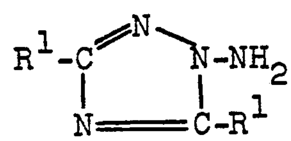

IIIb

und 4-Amino-4,2,1-triazole der Formel

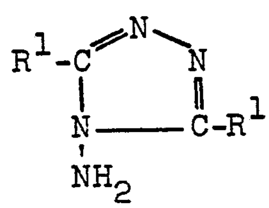

IIIc

und somit allgemein N-Amino-1,2,4-triazole der Formel

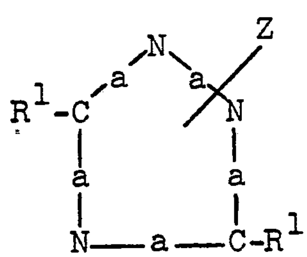

III,

worin die einzelnen Reste $R^1$ gleich oder verschieden sein können und jeweils ein Wasserstoffatom oder einen aliphatischen oder aromatischen Rest bedeuten, Z eine an einem Stickstoffatom sitzende Aminogruppe bedeutet, die beiden in α-Stellung zur Aminogruppe befindlichen Bindungen a jeweils eine Einfachbindung bedeuten, die beiden in ß-Stellung zur Aminogruppe befindlichen Bindungen a jeweils eine Doppelbindung bedeuten, und die verbleibende Bindung a in γ-Stellung zur Aminogruppe eine Einfachbindung bezeichnet, in Betracht.

Bevorzugte Ausgangsstoffe II und III und dementsprechend bevorzugte Endstoffe I sind solche, in deren Formeln die einzelnen Reste $R^1$ gleich oder verschieden sein können und jeweils ein Wasserstoffatom, einen Alkylrest mit 1 bis 10 Kohlenstoffatomen, insbesondere einen Alkylrest mit 1 bis 4 Kohlenstoffatomen, oder einen Phenylrest bedeuten. Die vorgenannten Reste $R^1$ können noch durch unter den Reaktionsbedingungen inerte Gruppen, z.B. Alkylgruppen oder Alkoxygruppen mit jeweils 1 bis 3 Kohlenstoffatomen, substituiert sein. Z.B. sind folgende Ausgangsstoffe II und III geeignet: 1-Amino-, 2-Amino-, 3-Amino-, 4-Amino-, 5-Amino-1,2,4-triazol; homologe in 3-Stellung oder 5-Stellung einfach oder in 3-Stellung und 5-Stellung zweifach gleich oder unterschiedlich durch die Methyl-, Äthyl-, Propyl-, Isopropyl-, Butyl-, Isobutyl-, sek.-Butyl-, Phenyl-, tert.-Butyl-gruppe substituierte Aminotriazole.

Die Ausgangsstoffe II oder III werden in stöchiometrischer Menge oder mit einem Überschuß an Alkohol, vorzugsweise in einer Menge von 3 bis 30, insbesondere von 5 bis 15 Äquivalenten (Mol, dividiert durch Zahl der Hydroxylgruppen im Molekül) Alkohol je Mol Ausgangsstoff II oder III, umgesetzt. Die Alkohole können aliphatische Mono- oder Polyalkohole sein. Bevorzugte Alkohole sind solche der Formel

$$R^3OH \quad V,$$

worin $R^3$ einen Alkylrest mit 1 bis 5 Kohlenstoffatomen oder den Rest $HO-R^4-$, in dem $R^4$ einen aliphatischen Rest, insbesondere einen Alkylenrest mit 2 bis 4 Kohlenstoffatomen bezeichnet, oder den Rest $R^5O-(R^4O)_n-R^4-$, worin die einzelnen Reste $R^4$ gleich oder verschieden sein können und für einen aliphatischen Rest, insbesondere einen Alkylenrest mit 2 bis 4 Kohlenstoffatomen stehen, und $R^5$ ein Wasserstoffatom oder einen aliphatischen Rest, insbesondere einen Alkylrest mit 1 bis 4 Kohlenstoffatomen, bezeichnet, und n für die Zahl 4, 3, 2 oder insbesondere 1 steht, bedeutet. Die vorgenannten Reste $R^3$, $R^4$ und $R^5$ können noch durch unter den Reaktionsbedingungen inerte Gruppen, z.B. Alkylgruppen oder Alkoxygruppen mit jeweils 1 bis 3 Kohlenstoffatomen, substituiert sein.

Als Alkohole V kommen z.B. Methanol, Äthanol, n- und i-Propanol, n-Butanol, Butanol-2, Isobutanol, Äthylenglykol, Diäthylenglykol, Methyläthylenglykol, n-Pentanol, Neopentylglykol, 1,3-Propylenglykol, 1,4-Gutandiol, 1,2-Propylenglykol, Triäthylenglykol, Diäthylenglykolmono-n-butyläther; oder entsprechende Gemische in Frage. Bevorzugt sind Äthanol, Isopropanol, Methyläthylenglykol, n-Propanol, Isobutanol.

Weiterhin verwendet man bei der Reaktion Nitrosierungsmittel, z.B. salpetrige Säure und Stoffe, die sich unter den Reaktionsbedingungen in salpetrige Säure umwandeln, wie nitrose Gase; Salze, bevorzugt Alkalisalze, der salpetrigen Säure, insbesondere Kaliumnitrit und Natriumnitrit; Ester der salpetrigen Säure, zweckmäßig Alkylnitrite. Im Falle der Verwendung von Alkylnitriten kann auf einen Zusatz von Alkohol ganz oder zweckmäßig teilweise verzichtet werden, da unter den Reaktionsbedingungen solche Nitrite eine Kombination von salpetriger Säure und dem entsprechenden Alkohol ersetzen können; in solchen

Fällen ist ein Verhältnis von 1 bis 5 Äquivalenten Alkohol je Mol Ausgangsstoff II oder III zweckmäßig. Vorzugsweise kommen bei den Estern Alkylnitrite mit 1 bis 6 Kohlenstoffatomen, z.B. Äthyl-, n-Propyl-, n-Isopropyl-nitrit, n-Butyl-, Isobutyl-, sek.-Butyl-, tert.-Butyl-, Amyl-, Isoamyl-nitrit und insbesondere Methylnitrit in Betracht. Unter nitrosen Gasen werden hier die als Nitrosierungsmittel bekannten Stickstoffoxide Stickstoffmonoxid, Stickstoffdioxid, Stickstofftetroxid, Distickstofftrioxid verstanden. Sie können einzeln oder zweckmäßig in einem entsprechenden Gemisch, vorteilhaft von Stickstoffmonoxid und Stickstoffdioxid, verwendet werden. Im allgemeinen kommen Mengen von 1,1 bis 5 Mol Alkylnitrit, Salpetrigsäureester und/oder nitrose Gase je Mol Ausgangsstoff II oder III, zweckmäßig 1,1 bis 2,7, insbesondere 1,1 bis 1,7 Mol Alkylnitrit bzw. Salpetrigsäureester oder 1,5 bis 5, insbesondere 2 bis 4 Mol $N_2O_3$ je Mol Ausgangsstoff II oder III, in Betracht. Den genannten Stickstoffoxiden bzw. Gasgemischen können noch unter den Reaktionsbedingungen inerte Gase, z.B. Stickstoff, zugemischt werden.

Ebenfalls kommen als Nitrosierungsmittel Glykolester der salpetrigen Säure in Frage. Diese Ester der salpetrigen Säure können in beliebiger Weise, zweckmäßig nach dem in der deutschen Offenlegungsschrift 21 44 420 beschriebenen Verfahren, durch Umsetzung von Glykolen oder Glykolderivaten mit salpetriger Säure oder Stickoxiden, hergestellt werden. Bevorzugte Ester von Glykolen und Glykolderivaten sind Mono- oder Diglykolester der salpetrigen Säure der Formel

$$ONO - R^6 - X \qquad IV,$$

worin $R^6$ den Rest $-R^7-O-$ oder den Rest $-(\underset{R^2}{\underset{|}{CH}}-CH_2O)_n-$ be-

deutet, $R^7$ einen aliphatischen Rest und $R^2$ ein Wasserstoffatom oder einen aliphatischen Rest bezeichnen, n für die Zahl 1, 2, 3 oder 4 steht und X die Gruppe -NO oder einen aliphatischen Rest bedeutet. Vorteilhaft bezeichnen $R^7$ einen Alkylenrest mit 3 bis 12, insbesondere 4 bis 9 Kohlenstoffatomen, $R^2$ ein Wasserstoffatom oder einen Alkylrest mit 1 bis 4 Kohlenstoffatomen, insbesondere die Methylgruppe, steht n für die Zahl 1, 2 oder 3 und bedeutet X die Gruppe -NO oder einen Alkylrest mit 1 bis 4 Kohlenstoffatomen. Die genannten Alkylreste und Alkylenreste können geradkettig oder verzweigt sein. Vorgenannte vorteilhafte Reste können noch durch unter den Reaktionsbedingungen inerte Gruppen, z.B. Alkoxygruppen, Alkylgruppen mit jeweils 1 bis 3 Kohlenstoffatomen, substituiert sein. Im allgemeinen kommen Mengen von 1,1 bis 5 Mol Monoglykolester je Mol Ausgangsstoff II oder III, zweckmäßig 1,1 bis 2,7, insbesondere 1,1 bis 2,2 Mol Monoglykolester je Mol Ausgangsstoff II oder III, in Betracht. Entsprechend wählt man Mengen von 0,55 bis 2,5, zweckmäßig 0,55 bis 1,35, insbesondere 0,55 bis 1,1 Mol Diglykolester je Mol Ausgangsstoff II oder III. Geeignete Ester IV sind beispielsweise Mono- bzw. Diester der salpetrigen Säure mit folgenden Verbindungen:

$CH_3O-CH_2CH_2-OH$

$C_2H_5O-CH_2CH_2-OH$

$C_3H_7O-CH_2CH_2-OH$

$C_4H_9O-CH_2CH_2-OH$

$HO-(CH_2CH_2O)_2H$,

$HO-(CH_2CH_2O)_3H$

$CH_3O(CH_2CH_2O)_2H$

$C_2H_5O(CH_2CH_2O)_2H$

$CH_3$
$CH_3O-CH_2-CH-OH$

$CH_3$
$CH_3O-(CH-CH_2O)_2H$

$CH_3$ $CH_3$
$HO-CH-CH_2OCH_2-CH-OH$,

$CH_3$
$C_2H_5O-(CH-CH_2O)_2H$

$CH_3COOCH_2CH_2OH$

$C_3H_7O(CH_2CH_2O)_2H$

$C_4H_9O(CH_2CH_2O)_2H$

$CH_3O(CH_2CH_2O)_3H$

$C_2H_5O(CH_2CH_2O)_3H$,

$C_3H_7O(CH_2CH_2O)_3H$,

$C_4H_9O(CH_2CH_2O)_3H$

$C_2H_5O(CH_2CH_2O)_4H$ ; Diglykolester, deren $R^6$ den Rest $-R^7-O-X$ die Gruppe $-NO$ und $R^7$ die Alkylenreste $-(CH_2)_3-$, $-(CH_2)_4-$, $-(CH_2)_5-$, $-(CH_2)_6-$, 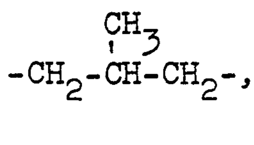, 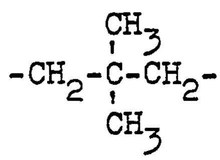,

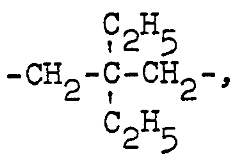, 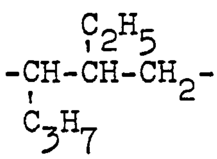, 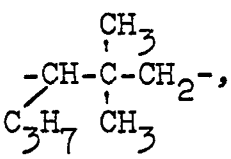, 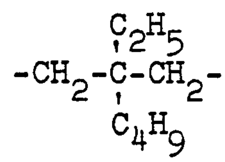 oder

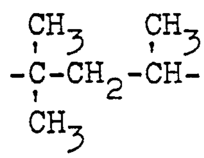 bedeuten.

Die Umsetzung wird unter Zusatz von Wasser, vorteilhaft in einer Menge von 5 bis 100, insbesondere von 15 bis 50 Mol Wasser je Mol Ausgangsstoff II oder III durchgeführt; das Wasser kann getrennt und/oder in Gestalt wäßriger Lösungen der Reaktionspartner, z.B. von wäßriger Säure, von wäßrigen Alkalinitritlösungen oder von Gemischen des Alkohols mit Wasser, zugesetzt werden. Das bei der Reaktion selbst gebildete Wasser wird in diesem Zusammenhang nicht als zugesetztes Wasser definiert und ist nicht in vorgenannten, vorteilhaften Wassermengen enthalten. Die Umsetzung wird in Gegenwart von Schwefelsäure, vorteilhaft in einer Menge von 1,5 bis 15, insbesondere von 2,5 bis 10 Äquivalenten Säure, bezogen auf Ausgangsstoff II oder III, durchgeführt. Die Säure kann in konzentrierter Form oder mit einem Lösungsmittel, insbesondere Wasser, angewendet werden.

Die Umsetzung wird bei einer Temperatur von mindestens $-20^{o}C$, in der Regel bei einer Temperatur von $-5^{o}C$ bis zur Siedetemperatur des Gemischs, zweckmäßig von 0 bis $200^{o}C$, vorzugsweise von 0 bis $50^{o}C$, drucklos oder unter Druck, kontinuierlich oder diskontinuierlich durchgeführt. In der Regel dienen Komponenten des Ausgangsgemischs, z.B. Wasser, Alkohol oder Säure, oder das gesamte Ausgangsgemisch als Lösungsmedium der Reaktion.

Die Reaktion kann wie folgt durchgeführt werden: Ein Gemisch von Ausgangsstoff II oder III, Alkohol, Nitrosierungsmittel, Säure und Wasser wird während 1,5 bis 5 Stunden bei der Reaktionstemperatur gehalten. Zweckmäßig läßt man das Nitrosierungsmittel, z.B. die wäßrige Natriumnitrit-Lösung, oder den Salpetrigsäureester zum Gemisch der Reaktionspartner zulaufen. Der Zulauf kann in einem weiten Bereich beliebig schnell erfolgen. Das Reaktionsende fällt häufig mit dem Ende des Zulaufs des Nitrierungsmittels zusammen. Aus dem Reaktionsgemisch wird der Endstoff in üblicher Weise, z.B. durch alkalische Einstellung des Gemischs, Filtration, Eiengen des Filtrats, Extraktion mit einem geeigneten Lösungsmittel wie Methanol oder Dioxan, Filtration und Destillation des Filtrats, abgetrennt.

Die nach dem Verfahren der Erfindung herstellbaren neuen Verbindungen sind wertvolle Ausgangsstoffe für die Herstellung von Pharmazeutika, Farbstoffen und Schädlingsbekämpfungsmitteln. Bezüglich der Verwendung wird auf vorgenannte Veröffentlichungen und DE-AS 17 95 249, DE-OS 27 20 949, DE-OS 24 01 715, DE-OS 24 31 407, DE-OS 27 13 777, DE-OS 24 06 665, verwiesen.

Die in den Beispielen genannten Teile bedeuten Gewichtsteile.

Beispiel 1

Man trägt 84 Teile 3-Amino-1,2,4-triazol in 360 Teile Isopropanol und 500 Teile Wasser ein und versetzt das Gemisch dann mit 210 Teilen konzentrierter Schwefelsäure (98 Gewichtsprozent). Bei 5°C gibt man eine Lösung von 133 Teilen Natriumnitrit in 300 Teilen Wasser innerhalb 30 Minuten zu, wobei sich Stickstoff entwickelt. Man rührt das Gemisch noch 3 Stunden bei Raumtemperatur nach, stellt bei dieser Temperatur mit 50-gewichtsprozentiger Natronlauge einen pH-Wert von 7,8 ein, kühlt das Gemisch auf 10°C ab und saugt ausgefallenes Natriumsulfat ab. Das Filtrat wird im Vakuum zur Trockene eingeengt und zweimal mit je 500 Teilen Methanol ausgekocht. Nach Filtration wird das Filtrat im Vakuum vom Lösungsmittel befreit. Man erhält 65,6 Teile 1,2,4-Triazol (95 % der Theorie Ausbeute) vom Schmelzpunkt 117°C.

Beispiel 2

Man trägt 84 Teile 4-Amino-1,2,4-triazol in ein Gemisch aus 360 Teilen Isopropanol und 400 Teilen Wasser ein und versetzt das Gemisch dann mit 210 Teilen konzentrierter Schwefelsäure (98 Gewichtsprozent). Bei 5°C gibt man eine Lösung von 133 Teilen Natriumnitrit in 300 Teilen Wasser innerhalb 30 Minuten zu, wobei sich Stickstoff entwickelt. Man rührt das Gemisch noch 3 Stunden bei Raumtemperatur nach, stellt bei dieser Temperatur mit 50-gewichtsprozentiger Natronlauge einen pH-Wert von 7,8 ein, kühlt das Gemisch auf 10°C ab und saugt ausgefallenes Natriumsulfat ab. Das Filtrat wird im Vakuum zur Trockene eingeengt und zweimal mit je 500 Teilen Dioxan ausgekocht. Nach Filtration wird das Filtrat im Vakuum vom Lösungsmittel befreit. Man erhält 64,9 Teile 1,2,4-Triazol (94 % der Theorie Ausbeute) vom Schmelzpunkt 117°C.

Beispiel 3

Man trägt 84 Teile 3-Amino-1,2,4-triazol in ein Gemisch aus 400 Teilen Äthanol und 500 Teilen Wasser ein und versetzt das Gemisch dann mit 210 Teilen konzentrierter Schwefelsäure (98 Gewichtsprozent). Bei 5°C gibt man eine Lösung von 133 Teilen Natriumnitrit in 300 Teilen Wasser innerhalb 30 Minuten zu, wobei sich Stickstoff entwickelt. Man rührt das Gemisch noch 3 Stunden bei Raumtemperatur nach, stellt bei dieser Temperatur mit 50-gewichtsprozentiger Natronlauge einen pH-Wert von 7,8 ein, kühlt auf 10°C ab und saugt ausgefallenes Natriumsulfat ab. Nach Waschen mit 100 Teilen Äthanol wird das Filtrat im Vakuum zur Trockene eingeengt und der erhaltene Rückstand zweimal mit je 500 Teilen Dioxan ausgekocht. Nach Filtration wird das Filtrat im Vakuum vom Lösungsmittel befreit. Man erhält 60,7 Teile 1,2,4-Triazol (88 % der Theorie Ausbeute) vom Schmelzpunkt 117°C.

Beispiel 4

Man trägt 49 Teile 3-Amino-5-methyl-1,2,4-triazol in ein Gemisch aus 400 Teilen Isopropanol und 500 Teilen Wasser ein und versetzt anschließend mit 105 Teilen konzentrierter Schwefelsäure (98 Gewichtsprozent). Bei 5°C gibt man eine Lösung von 67 Teilen Natriumnitrit in 150 Teilen Wasser innerhalb 30 Minuten zu, wobei sich Stickstoff

entwickelt. Man rührt das Gemisch noch 3 Stunden bei Raumtemperatur nach, stellt bei dieser Temperatur mit 50-gewichtsprozentiger Natronlauge einen pH-Wert von 7,8 ein, kühlt auf 0°C und saugt ausgefallenes Natriumsulfat ab. Nach Waschen mit 100 Teilen Isopropanol wird das Filtrat im Vakuum vom Lösungsmittel befreit und der erhaltene Rückstand zweimal mit je 300 Teilen Dioxan ausgekocht. Die vereinigten Dioxanlösungen werden bis zur Trockene im Vakuum eingeengt. Man erhält 39 Teile 3-Methyl-1,2,4-triazol (94 % der Theorie) vom Schmelzpunkt 93°C.

Beispiel 5

Man trägt 160 Teile 3-Amino-5-phenyl-1,2,4-triazol in ein Gemisch aus 400 Teilen Isopropanol und 500 Teilen Wasser ein und versetzt mit 210 Teilen konzentrierter Schwefelsäure (98 Gewichtsprozent). Bei 5°C gibt man eine Lösung von 133 Teilen Natriumnitrit in 300 Teilen Wasser innerhalb 30 Minuten zu, wobei sich Stickstoff entwickelt. Man rührt das Gemisch noch 3 Stunden bei Raumtemperatur nach, stellt bei dieser Temperatur mit 50-gewichtsprozentiger Natronlauge einen pH-Wert von 7,8 ein, kühlt auf 5°C ab und saugt ausgefallenes Natriumsulfat ab. Nach Waschen mit 150 Teilen Isopropanol wird das Filtrat im Vakuum vom Lösungsmittel befreit und der erhaltene Rückstand einmal mit 500 Teilen Dioxan ausgekocht. Nach Abtrennung des Dioxans erhält man 100 Teile 3-Phenyl-1,2,4-triazol (69 % der Theorie) vom Schmelzpunkt 119°C.

Beispiel 6

Man trägt 84 Teile 3-Amino-1,2,4-triazol in 500 Teile Glykolmonoäthyläther ein und versetzt bei 25°C mit 800 Teilen 50-gewichtsprozentiger Schwefelsäure. Bei 5°C gibt man eine Lösung aus 90 Teilen Natriumnitrit in 150 Teilen Wasser innerhalb 30 Minuten zu. Man rührt 3 Stunden bei 25°C nach, stellt dann bei dieser Temperatur mit 50-gewichtsprozentiger Natronlauge einen pH-Wert von 7,8 ein, kühlt auf 10°C, saugt ausgefallenes Natriumsulfat ab und wäscht mit 200 Teilen Isopropanol nach. Das Filtrat wird im Vakuum vom Lösungsmittel befreit und der Rückstand zweimal mit je 300 Teilen Dioxan ausgekocht. Die vereinigten Dioxanlösungen werden bis zur Trockene im Vakuum eingeengt. Der Endstoff wird mit Äther gewaschen und getrocknet. Man erhält 49 Teile 1,2,4-Triazol (71 % der Theorie) vom Schmelzpunkt 116°C.

Patentanspruch

Verfahren zur Herstellung von in 1-, 2- und 4-Stellung unsubstituierten, in 3- und/oder 5-Stellung gegebenenfalls durch jeweils einen aliphatischen oder aromatischen Rest substituierten 1,2,4-Triazolen durch Desaminierung von Aminotriazolen unter Verwendung von Nitrosierungsmitteln und Säure, dadurch gekennzeichnet, daß man gegebenenfalls in 3- und/oder 5-Stellung durch jeweils einen aliphatischen oder aromatischen Rest substituierte Amino-1,2,4-triazole mit aliphatischen Alkoholen und Nitrosierungsmitteln in Gegenwart von Wasser und Schwefelsäure umsetzt.